# EUROPEAN PATENT SPECIFICATION

(11) **EP 2 221 303 B1**
(45) Date of publication and mention of the grant of the patent: **07.01.2015**
(21) Application number: 10164785.7
(22) Date of filing: 05.11.2008
(51) Int. Cl.: C07D 413/04, C07D 498/04, C08G 73/00, H01M 8/10, C08J 5/22

(54) **Benzoxazine-based monomer, polymer thereof, electrode for fuel cell including the polymer, electrolyte membrane for fuel cell including the polymer, and fuel cell using the electrode**
Auf Benzoxazin basierendes Monomer, entsprechendes Polymer, Elektrode für eine Brennstoffzelle, die das Polymer enthält, Elektrolytmembran für eine Brennstoffzelle, die das Polymer enthält, und Brennstoffzelle, die die Elektrode einsetzt
Monomère à base de benzoxazine, polymère correspondant, électrode pour pile à combustible incluant le polymère, membrane électrolyte pour pile à combustible incluant le polymère, et pile à combustible l'utilisant

(30) Priority: 06.11.2007 KR 20070112750; 10.10.2008 KR 20080099549
(43) Date of publication of application: 25.08.2010
(62) Divisional of application: 08168404.5
(73) Proprietor: Samsung Electronics Co., Ltd., Suwon-si, Gyeonggi-do, 443-742 (KR)
(72) Inventor: Choi, Seongwoo, 449-712, Gyeonggi-do (KR); Park, Jungock, 449-712, Gyeonggi-do (KR)
(74) Representative: Zijlstra, Robert Wiebo Johan

(56) References cited:
- EP-A- 1 760 110
- US-A1- 2007 184 323

## Description

One or more embodiments of the present invention relate to an electrode for a fuel cell and a fuel cell employing the same.

Fuel cells, which use a polymer electrolyte membrane as an electrolyte, operate at a relatively low temperature and can also be small in size, and thus they are expected to be used as light sources in electric vehicles or distributed generation systems for homes. As a polymer electrolyte membrane used in polymer electrolyte fuel cells, a perfluorocarbonsulfonic acid-based polymer membrane available from Nafion (registered trademark) is used.

However, such polymer electrolyte membranes need water for exhibiting proton conduction abilities, and thus the polymer electrolyte membranes need humidifying. In addition, to enhance cell system efficiencies, it is necessary to operate polymer electrolyte membranes at a high temperature of at least 100°C. However, the moisture in polymer electrolyte membranes is evaporated at this temperature, and polymer electrolyte membranes can not function as a solid electrolyte.

To address those problems in the art, non-humidified electrolyte membranes which can operate at a high temperature of at least 100°C under nonhumidified conditions have been developed. For example, US Patent No. 5,525,436 discloses polybenzimidazole doped with a phosphoric acid, and the like as a material constituting non-humidified electrolyte membranes.

In addition, in cells which operate at a low temperature, such as the cells using a perfluorocarbonsulfonic acid-based polymer membrane, to prevent gas diffusion in electrodes due to water (formation water) that is produced as electricity is generated in an electrode, particularly a cathode, electrodes using polytetrafluoroethylene (PTFE) as a waterproof agent to have hydrophobic properties have been widely used (For example, Japanese Patent Laid-Open Publication No. hei 05-283082).

In addition, phosphoric acid type fuel cells operating at a high temperature of 150 to 200°C use a liquid phosphoric acid as an electrolyte. However, a large amount of the liquid phosphoric acid exists in electrodes which interferes with gas diffusion. Therefore, an electrode catalyst layer which is formed by adding polytetrafluoroethylene (PTFE) as a waterproof agent to an electrode catalyst, and which can prevent fine pores in electrodes from being clogged by a phosphoric acid, has been used.

In addition, in fuel cells using a polybenzimidazole (PBI) electrolyte membrane which retains a phosphoric acid as a nonhumidified electrolyte at a high temperature, to reduce contact between electrodes and the electrolyte membrane, a method of impregnating electrodes with a liquid phosphoric acid has been tried and a method of increasing a loading amount of metal catalysts has been tried. However, the fuel cells do not exhibit improved properties, and thus there is a need for improvement.

In addition, in the case of supplying air to a cathode when a solid polymer electrolyte doped with phosphoric acid is used, the fuel cell requires an aging time of about 1 week even if the composition of the cathode is optimized. By supplying oxygen to the cathode instead of air, performances of the cathode can be improved and the aging time can also be reduced. However, the supply of oxygen to the cathode is an obstacle in realizing widespread use of the cathode. In addition, the polymer electrolyte membrane formed of PBI does not have satisfactory mechanical properties and chemical stability at a high temperature and capability of retaining a phosphoric acid.

European patent application with publication number EP 1 760 110 A1 discloses a crosslinked object of a polybenzoxazine-based compound formed of a polymerized resultant of a first monofunctional benzoxazine-based monomer or a second multifunctional benzoxazine-based monomer with a crosslinkable compound, an electrolyte membrane including the crosslinked object, a method of preparing the electrolyte membrane, and a fuel cell employing the electrolyte membrane including the crosslinked object.

US patent application with publication number US 2007/184323 A1 discloses electrolyte membrane including a cross-linked reaction product of a benzoxazine monomer and a cross-linkable compound. The electrolyte membrane is impregnated with 300 to 600 parts by weight of phosphoric acid based on 100 parts by weight of the electrolyte membrane, and has a yield strain 0.5% or less, and a yield stress 0.3 Mpa or less. The cross-linked material can be obtained by a simple polymerization process by combining a benzoxazine monomer and a crosslinkable compound and by using heat instead of using a polymerization initiator or a cross-linking agent.

One or more embodiments of the present invention include a benzoxazine-based monomer having excellent thermal resistance and high phosphoric acid resistance, a polymer thereof, an electrode for a fuel cell including the polymer, an electrolyte membrane for a fuel cell including the polymer, and a fuel cell using the electrode.

Additional aspects and/or advantages will be set forth in part in the description which follows and, in part, will be apparent from the description, or may be learned by practice of the invention.

To achieve the above and/or other aspects and advantages, one or more embodiments of the present invention may include a benzoxazine-based monomer represented by Formula 5 or Formula 12 below: 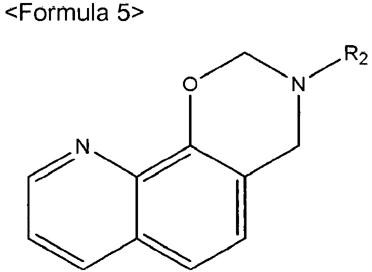 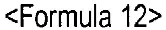 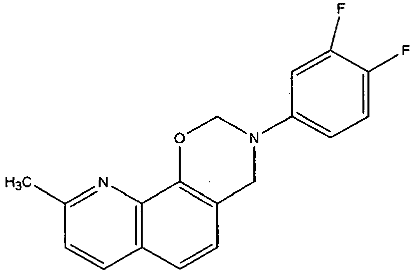 wherein R₂ is a halogen atom, a halogenated C₁-C₂₀ alkyl group, a halogenated C₁-C₂₀ alkoxy group, a halogenated C₂-C₂₀ alkenyl group, a halogenated C₂-C₂₀ alkynyl group, a halogenated C₆-C₂₀ aryl group, a halogenated C₆-C₂₀ aryloxy group, a halogenated C₂-C₂₀ heteroaryl group, a halogenated C₂-C₂₀ heteroaryloxy group, a halogenated C₄-C₂₀ cycloalkyl group, or a halogenated C₂-C₂₀ heterocyclic group.

To achieve the above and/or other aspects and advantages, one or more embodiments of the present invention may include a polymer of benzoxazine-based monomer which is a polymerization product of the benzoxazine-based monomer described above or a polymerization product between the benzoxazine-based monomer described above and a crosslinkable compound.

To achieve the above and/or other aspects and advantages, one or more embodiments of the present invention may include an electrode for a fuel cell, the electrode comprising a catalyst layer comprising the polymer of the benzoxazine-based monomer described above.

To achieve the above and/or other aspects and advantages, one or more embodiments of the present invention may include an electrolyte membrane for a fuel cell, comprising the polymer of the benzoxazine-based monomer described above.

To achieve the above and/or other aspects and advantages, one or more embodiments of the present invention may include a fuel cell including the electrode for a fuel cell. An electrolyte membrane in the fuel cell may comprise the polymer of the benzoxazine-based monomer described above.

To achieve the above and/or other aspects and advantages, one or more embodiments of the present invention may include a fuel cell including the electrolyte membrane for a fuel cell.

These and/or other aspects and advantages will become apparent and more readily appreciated from the following description of the embodiments, taken in conjunction with the accompanying drawings of which:
FIGS. 1 through 7 are graphs respectively showing nuclear magnetic resonance (NMR) spectra of benzoxazine-based monomers prepared in Synthesis Examples 1 through 7;
FIG. 8 is a graph showing NMR spectrum of a polymer of a benzoxazine-based monomer prepared in Synthesis Example 8;
FIG. 9 is a graph showing thermogravimetric analysis (TGA) results of 3HP-34DFA prepared in Synthesis Example 5 and t-BuPh-a prepared in Reference Example 1;
FIG. 10 is a graph showing a change in voltage according to time of fuel cells manufactured in Example 1 and Comparative Example 1;
FIG. 11 is a graph showing a change in cell voltage according to current density of fuel cells manufactured in Examples 1 and 2 and Comparative Example 1;
FIG. 12 is a graph showing cell voltage according to current density of a fuel cell manufactured in Example 7;
FIG. 13 is a graph showing evaluation results of durability of an electrolyte membrane prepared in Example 7;
FIGS. 14 and 15 are graphs showing measurement results of conductivity according to temperature and phosphoric acid doping level of electrolyte membrane prepared in Examples 7 and 8;
FIG. 16 is a graph showing a change in cell voltage and resistance according to current density of an electrolyte membrane prepared in Example 8;
FIG. 17 is a graph showing a change in cell voltage and resistance according to current density of an electrolyte membrane prepared in Example 9;
FIG. 18 is a graph showing a change in cell voltage and resistance according to current density of an electrolyte membrane prepared in Example 10;
FIG. 19 is a graph showing evaluation results of thermal stability of electrolyte membrane prepared in Examples 7 and 8; and
FIG. 20 is a graph showing cell voltage according to current density of fuel cells manufactured in Example 11 and Comparative Example 2.

The present application is a divisional application from European patent application no. 08168404.5. It is noted for the sake of clarity that the compounds of Formulas 1-4, 6-9, 10 and 13 and products including those compounds do not form part of the invention claimed in this divisional application.

Reference will now be made in detail to embodiments, examples of which are illustrated in the accompanying drawings, wherein like reference numerals refer to the like elements throughout. In this regard, the present invention may be embodied in many different forms and should not be construed as being limited to the embodiments set forth herein. Accordingly, embodiments are merely described below, by referring to the figures, to explain aspects of the present invention.

A benzoxazine-based monomer represented by Formula 1 below may be provided: 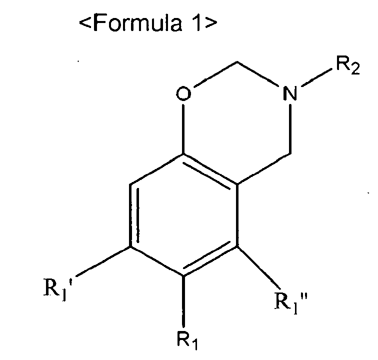 wherein at least one of R₁, R₁^{'}, R₁^{"}, and R₂ is a halogen atom, a halogenated C₁-C₂₀ alkyl group, a halogenated C₁-C₂₀ alkoxy group, a halogenated C₂-C₂₀ alkenyl group, a halogenated C₂-C₂₀ alkynyl group, a halogenated C₆-C₂₀ aryl group, a halogenated C₆-C₂₀ aryloxy group, a halogenated C₂-C₂₀ heteroaryl group, a halogenated C₂-C₂₀ heteroaryloxy group, a halogenated C₄-C₂₀ cycloalkyl group, or a halogenated C₂-C₂₀ heterocyclic group, and another one of R₁, R₁^{'}, R₁^{"}, and R₂ is a substituted or unsubstituted non-halogenated C₂-C₂₀ heterocyclic group; and any remaining ones of R₁, R₁', R₁" and R₂ are hydrogen.

The term "halogenated" used in Formula 1 refers to substitution with a halogen atom such as fluorine, chlorine, or iodine.

In Formula 1, at least one of R₁, R₁^{'}, R₁^{"}, and R₂ may be fluorine, a fluorinated C₁-C₂₀ alkyl group, a fluorinated C₆-C₂₀ aryl group, a fluorinated C₂-C₂₀ heteroaryl group, a fluorinated C₂-C₂₀ heteroaryloxy group, a fluorinated C₄-C₂₀ cycloalkyl group, or a fluorinated C₂-C₂₀ heterocyclic group, and the other one of R₁, R₁^{'}, R₁^{"}, and R₂ may be a nitrogen-containing C₃-C₆ heterocyclic group derived from tertiary amine such as pyridine.

In addition, in Formula 1, the nitrogen-containing C₃-C₆ heterocyclic group is one selected from the groups represented by the following formulae:

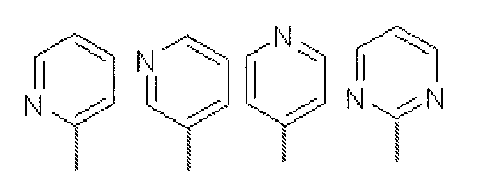

The fluorinated C₆-C₂₀ aryl group is one selected from the grounds represented by the following formulae:

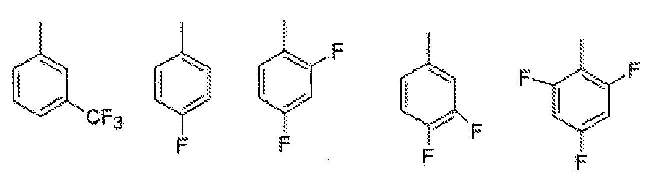

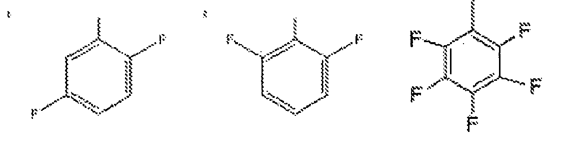

In Formula 1, at least one of R₁, R₁', and R₁" may be fluorine, a fluorinated C₁-C₂₀ alkyl group, a fluorinated C₆-C₂₀ aryl group, a fluorinated C₂-C₂₀ heteroaryl group, a fluorinated C₂-C₂₀ heteroaryloxy group, a fluorinated C₄-C₂₀ cycloalkyl group, or a fluorinated C₂-C₂₀ heterocyclic group, and R₂ may be a nitrogen-containing C₃-C₆ heterocyclic group.

The benzoxazine-based monomer of Formula 1 may be one of the compounds represented by Formulae 6 through 9:

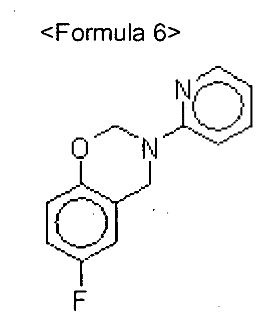

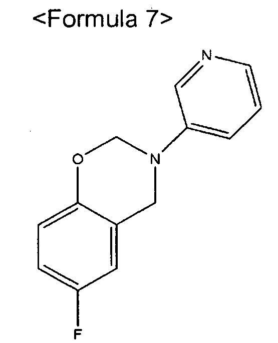

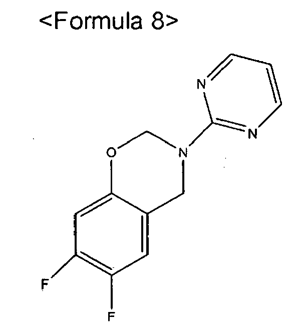

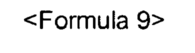

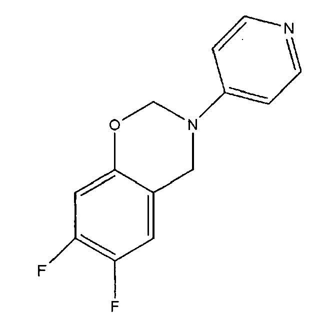

An embodiment of the present invention provides a benzoxazine-based monomer represented by Formula 5 below. Pyrido-oxazine-based monomers represented by any of Formulae 2-4 do not form part of the present invention. 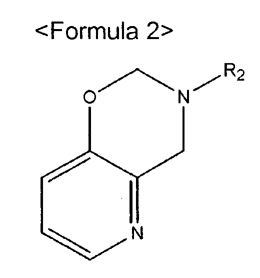 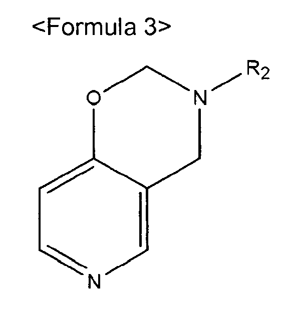 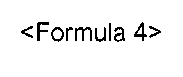 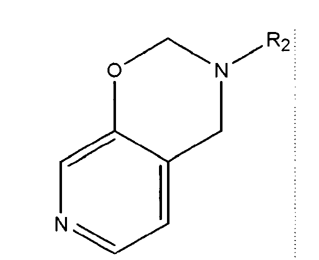 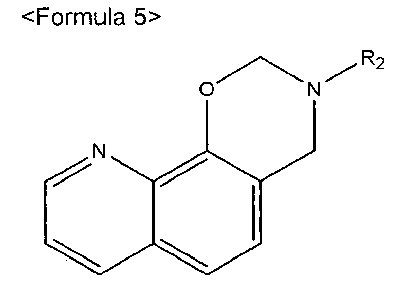 wherein R₂ is a halogen atom, a halogenated C₁-C₂₀ alkyl group, a halogenated C₁-C₂₀ alkoxy group, a halogenated C₂-C₂₀ alkenyl group, a halogenated C₂-C₂₀ alkynyl group, a halogenated C₆-C₂₀ aryl group, a halogenated C₆-C₂₀ aryloxy group, a halogenated C₂-C₂₀ heteroaryl group, a halogenated C₂-C₂₀ heteroaryloxy group, a halogenated C₄-C₂₀ cycloalkyl group, or a halogenated C₂-C₂₀ heterocyclic group.

R₂ may be one of the groups represented by the following formulae.

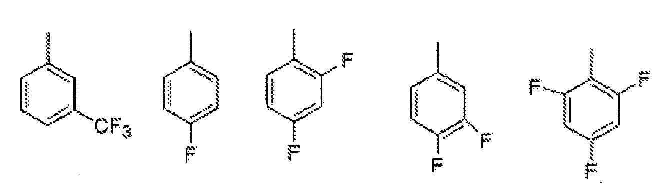

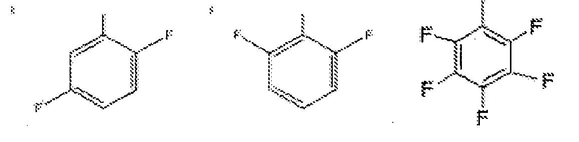

The benzoxazine-based or pyrido-oxazine-based monomer represented by one of Formulae 2-5 may be one selected from compounds represented by Formulae 10 through 13 below.

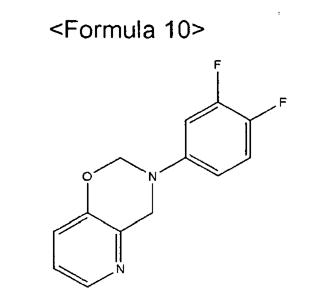

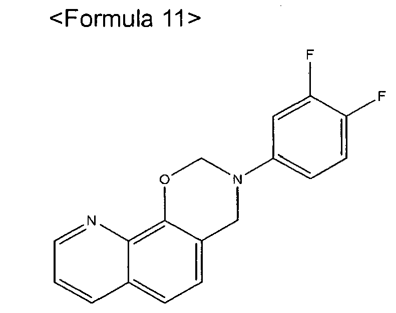

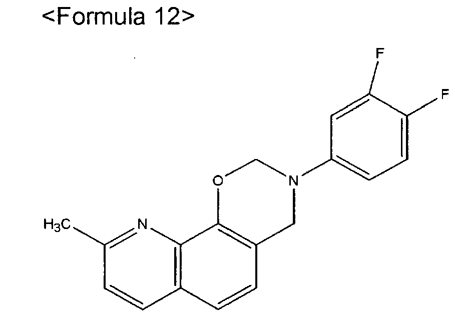

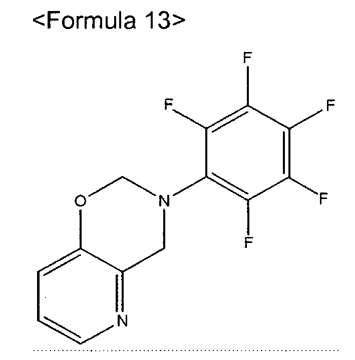

A polymer of benzoxazine-based monomer according to an embodiment of the present invention may be obtained by polymerization of the benzoxazine-based monomer described above or polymerization between the benzoxazine-based monomer described above and a crosslinkable compound.

The benzoxazine-based monomer represented by one of Formulae 6-9 has fluorine or a fluorine-containing functional group as R₁ and a pyridyl group as R₂. Due to these structural properties, the benzoxazine-based monomer and a polymer thereof contain the fluorine functional group, thereby having improved oxygen transmission and contain the pyridyl group, thereby having excellent heat resistance and resistance to phosphoric acid.

In addition, the benzoxazine-based monomer represented by one of Formulae 11 or 12 and a polymer thereof have the same structural properties as those of the benzoxazine-based monomer represented by one of Formulae 6-9. That is, the benzoxazine-based monomer represented by one of Formulae 11 or 12 and a polymer thereof contain a fluorine group, thereby having improved thermal stability at a high temperature and contain a pyridine-based amine structure, thereby having improved capability of retaining acid.

An electrode for a fuel cell includes a catalyst layer comprising the polymer of the benzoxazine-based monomer of Formula 1.

The catalyst layer includes a catalyst.

The polymer of the benzoxazine-based monomer represented by Formula 1 is used as a binder of the electrode, and in particular, can act as a binder. Thus, a commonly used binder is not necessary for the electrode.

The electrode of the present invention contains the polymer of the benzoxazine-based monomer as described above, and thus the transmission of oxygen into the electrode is increased, and thermal resistance and resistance to phosphoric acid of the electrode are improved. As a result, doped phosphoric acid can easily be wet into the electrode.

The benzoxazine-based monomer of Formula 1 may be prepared according to Reaction Scheme 1 below.

Referring to Reaction Scheme 1, the benzoxazine-based monomer of Formula 2 can be prepared by heating a phenol compound (A), p-formaldehyde (B) and an amine compound (C) without a solvent or adding a solvent to A, B and C and then refluxing the mixture, and thereafter working up the resultant: 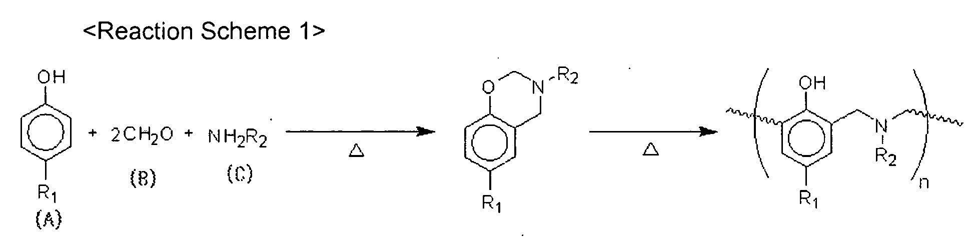 wherein R₁ and R₂ are the same as defined in Formula 1, and R₁' and R₁" are each independently hydrogen in the compound of Formula 1 in Reaction Scheme 1.

In the case of adding the solvent to the phenol compound (A), p-formaldehyde (B) and the amine compound (C), the solvent used may be 1,4-dioxane, chloroform, dichloromethane, THF, or the like. The heating temperature is adjusted to be within a range of temperatures at which the solvent used can be refluxed.

The benzoxazine-based monomer represented by Formula 5 and polymers thereof can be prepared in the same manner as in Reaction Scheme 1, except that a phenol compound corresponding to the phenol compound (A) (for example, 3-hydroxypyridine, 4-hydroxypyridine, 5-hydroxypyridine, or the like) is used.

The electrode for a fuel cell, which is finally obtained according to an embodiment of the present invention, does not contain the benzoxazine-based monomer represented by Formula 5, but contains the polymer thereof. The benzoxazine-based monomer of Formula 5 is polymerized during a drying process of a composition for forming an electrode active material layer in the formation of the electrode and/or while a fuel cell including the electrode operates, thereby being converted to the polymer thereof.

When the polymer of the benzoxazine-based monomer represented by Formula 5 of the present invention is used in forming an electrode for a fuel cell, oxygen transmission is improved even by supplying air to a cathode, and wettability of phosphoric acid (H₃PO₄) in an electrode and thermal stability can be improved. Therefore, a fuel cell employing the electrode and an electrolyte membrane can operate at a high temperature under nonhumidified condition, can have enhanced thermal stability, and can exhibit improved electricity generation performance.

The amount of the polymer of the benzoxazine-based monomer represented by Formula 5 may be in the range of 0.001 to 0.65 parts by weight, and specifically in the range of 0.01 to 0.05 parts by weight based on 1 part by weight of the catalyst.

When the polymer of the benzoxazine-based monomer represented by Formula 5 is less than 0.001 parts by weight based on 1 part by weight of the catalyst, wettability of phosphoric acid in an electrode may not be sufficiently improved. On the other hand, when the amount of the polymer of the benzoxazine-based monomer represented by Formula 5 is greater than 0.65 parts by weight based on 1 part by weight of the catalyst, flooding of phosphoric acid may be facilitated.

The catalyst may be platinum alone, or an alloy or mixture of platinum and at least one metal selected from the group consisting of gold, palladium, rhodium, iridium, ruthenium, tin, molybdenum, cobalt, and chrome. Alternatively, the catalyst may be a support catalyst in which the catalyst metal is loaded on a carbonaceous support. In particular, the catalyst may be a catalyst metal including at least one of Pt, PtCo, and PtRu, or a support catalyst in which the catalyst metal is loaded on a carbonaceous support.

The electrode may further include a binder which can be conventionally used in the preparation of an electrode for a fuel cell.

The binder may be at least one selected from the group consisting of polyvinylidenefluoride, polytetrafluoroethylene (PTFE), tetrafluoroethylene-hexafluoroethylene copolymer, and perfluoroethylene. The amount of the binder may be in the range of 0.001 to 0.5 parts by weight based on 1 part by weight of the catalyst. When the amount of the binder is less than 0.001 parts by weight based on 1 part by weight of the catalyst, wettability of phosphoric acid in an electrode may not be sufficiently improved. On the other hand, when the amount of the binder is greater than 0.5 parts by weight based on 1 part by weight of the catalyst, flooding of phosphoric acid may be facilitated.

A method of preparing an electrode for a fuel cell will now be described.

First, a catalyst is dispersed in a solvent to obtain a dispersion. The solvent used is N-methylpyrolidone (NMP), dimethylformamide (DMAc), or the like, and the amount of the solvent is in the range of 1 to 10 parts by weight based on 1 part by weight of the catalyst.

A mixture of the benzoxazine-based monomer of Formula 1, a binder, and a solvent is added to the dispersion and mixed together, and then the resultant is stirred.

The mixture may further include a crosslinkable compound, if necessary. Examples of the crosslinkable compound include at least one selected from polybenzimidazole (PBI), polybenzimidazole-base complexes, polybenzthiazoles, polybenzoxazoles, and polyimides. The amount of crosslinkable compound may be in the range of 5-95 parts by weight based on 100 parts by weight of the benzoxazine-based monomer of Formula 1.

The solvent is N-methylpyrolidone (NMP), dimethylacetamide (DMAc), or the like.

The resultant is coated on the surface of a carbon support to prepare an electrode. Herein, the carbon support may be fixed on a glass substrate in order to easily coat the resultant thereon. The coating method is not particularly limited, but, may be coating using a doctor blade, bar coating, screen printing, or the like.

The coated resultant is dried at a temperature in the range of 20 to 150°C, wherein the drying process is performed for removing the solvent. The drying time is dependent on the drying temperature, and is in the range of 10 to 60 minutes. The drying process may be performed at room temperature for 1 hour, at 60°C for at least 15 minutes, at 80°C for at least 10 minutes, and at 120°C for at least 10 minutes.

In addition, the catalyst layer of the electrode may further include at least one proton conductor selected from a phosphoric acid and a C₁-C₂₀ organic phosphonic acid. The amount of the proton conductor is in the range of 10 to 1,000 parts by weight based on 100 parts by weight of the total weight of the electrode. The concentration of the acid used is not particularly limited. However, in the case of a phosphoric acid, 85 wt% of an aqueous phosphoric acid solution is used, and the impregnation time of the phosphoric acid is in the range of 2.5 to 14 hours at 80°C.

An electrolyte membrane according to an embodiment of the present invention comprises a polymer of benzoxazine-based monomer which is a polymerization product of the benzoxazine-based monomer represented by Formula 5 or a polymerization product between the benzoxazine-based monomer represented by Formula 5 and a crosslinkable compound.

The crosslinkable compound may be the same as that described in the formation of the electrode. In particular, examples of the crosslinkable compound include at least one selected from polybenzimidazole (PBI), polybenzimidazole-base complexes, polybenzthiazoles, polybenzoxazoles and polyimides. The polybenzimidazole-base complexes are disclosed in Korean Patent No. 2007-102579 applied by the present applicant.

When the crosslinkable compound is polybenzimidazole or a polybenzimidazole-base complex, the electrolyte membrane comprises a crosslinked object of a polybenzoxazine-based compound which is obtained by curing a thermosetting resin, polybenzoxazine with a thermoplastic resin, polybenzimidazole or a polybenzimidazole-base complex.

In addition, as described above, the electrolyte membrane comprises the crosslinked object of a polybenzoxazine-based compound which is a polymerization product between the benzoxazine-based monomer of Formula 1 and a crosslinkable compound, and thus problems occurring when an electrolyte membrane formed of polybenzimidazole alone is used, for example, a pin-hole phenomenon caused by deficient mechanical and chemical stabilities at a high temperature can be addressed. In addition, when the electrode includes a polymer of a halogen-containing benzoxazine-based monomer, in particular, a fluorine-containing benzoxazine-based monomer, as described above, the transmission of oxygen into the electrode increases and the amount of dissolved oxygen in the electrode increases, and thus activation time is decreased.

A fuel cell according to an embodiment of the present invention comprises optimized electrolyte membrane forming material and/or electrode forming material, thereby having maximized cell performance.

Hereinafter, an electrolyte membrane and a method of preparing the electrolyte membrane will be described. When an electrolyte membrane is prepared only using the benzoxazine-based monomer of Formula 1, the preparation process is the same as those described herein, except that the crosslinkable compound was not used.

As a first method, the benzoxazine-based monomer represented by Formula 1 is blended with a crosslinkable compound, and the mixture is cured at a temperature in the range of 50 to 250°C, and preferably 80 to 220°C. The cured mixture is impregnated with a proton conductor such as an acid to prepare an electrolyte membrane.

The amount of the crosslinkable compound may be in the range of 5 to 95 parts by weight based on 100 parts by weight of the benzoxazine-based monomer of Formula 1.

When the amount of the crosslinkable compound is less than 5 parts by weight based on 100 parts by weight of the benzoxazine-based monomer of Formula 1, the electrolyte membrane is not impregnated with a phosphoric acid, and thus, proton conductivity of the electrolyte membrane is reduced. On the other hand, when the amount of the crosslinkable compound is greater than 95 parts by weight based on 100 parts by weight of the benzoxazine-based monomer of Formula 1, the crosslinked object is partially dissolved in polyphosphoric acid due to the presence of excessive phosphoric acid.

As a second method, an electrolyte membrane is formed using a mixture of the benzoxazine-based monomer represented by Formula 1 and a crosslinkable compound.

The formation of the electrolyte membrane may be performed by a tape casting method, or a conventional coating method. The conventional coating method may be a method in which the mixture is cast on a support using a doctor blade. Herein, a doctor blade with a 250 to 500µm gap is used.

When the casting method using a doctor blade is used, the process of forming the electrolyte membrane further includes separating the electrolyte membrane from the support, between the time when curing of the mixture occurs and the time when impregnation of the resultant with acid occurs. When it is time to perform the process of separating the electrolyte membrane from the support, the mixture is immersed in distilled water with a temperature in the range of 60 to 80°C.

The support can be any support which can support an electrolyte membrane, for example, a glass substrate, a polyimide film, and the like. When the tape casting method is used, a tape cast membrane is separated from a support such as polyethylenetherephthalate before being cured, and then put into an oven.

In addition, when a membrane is formed by the tape casting method using a mixture of a benzoxazine-based monomer and polybenzimidazole, a process of filtering the mixture may be further performed.

The tape cast membrane is cured by heat treatment, and then is impregnated with a proton conductor such as acid to form an electrolyte membrane.

Nonrestrictive examples of the proton conductor include a phosphoric acid, a phosphonic acid, a C₁-C₂₀ organic phosphonic acid, and the like. The C₁-C₂₀ organic phosphonic acid may be ethyl phosphonic acid or methyl phosphonic acid.

The amount of the proton conductor is in the range of 300 to 1,000 parts by weight based on 100 parts by weight of the total weight of the electrolyte membrane. The concentration of the acid used is not particularly limited. However, in the case of a phosphoric acid, 85 wt% of an aqueous phosphoric acid solution is used, and the impregnation time of the phosphoric acid is in the range of 2.5 to 14 hours at 80°C.

A method of preparing a fuel cell using the electrode for a fuel cell according to an embodiment of the present invention will now be described.

Any electrolyte membrane that is commonly used in the preparation of fuel cells can be used herein. Alternatively, an electrolyte membrane including a crosslinked product of polybenzoxazine-based compounds that is prepared by polymerization of the benzoxazine-based monomer represented by Formula 1 and a crosslinkable compound can also be used.

In particular, when the electrolyte membrane including the crosslinked product of polybenzoxazine-based compounds according to an embodiment of the present invention is used, contact resistance between the electrode and electrolyte membrane is decreased due to an improvement in compatibility, and thus cell performance of the fuel cell can be maximized.

For example, the electrolyte membrane that is commonly used in a fuel cell may be a polybenzimidazole electrolyte membrane, a polybenzoxazine-polybenzimidazole copolymer electrolyte membrane, a polytetrafluoroethylene (PTFE) porous membrane, or the like.

A method of manufacturing a membrane-electrode assembly for a fuel cell is as follows. The term "membrane and electrode assembly (MEA)" used herein refers to a structure in which an electrode, comprising a catalyst layer and a diffusion layer, is deposited on both surfaces of the electrolyte membrane.

The MEA may be formed by positioning the electrode including the catalyst layer for an electrode described above at both sides of the electrolyte membrane prepared by the process described above, joining them all together at a high temperature and a high pressure, and then joining a fuel diffusion layer to the catalyst layers.

Herein, the joining is performed under a pressure in the range of 0.1 to 3 ton/cm², and particularly about 1 ton/cm², in a state reached when the MEA is heated up to a temperature that softens the electrolyte membrane.

Next, a bipolar plate is disposed on each side of the membrane-electrode assembly to manufacture a fuel cell. The bipolar plate has grooves used for supplying fuel, and functions as a current collector.

The use of the fuel cell of the present invention is not particularly limited. However, the fuel cell may be preferably used as a polymer electrolyte membrane (PEM) fuel cell.

The substituents used herein are defines as follows.

The C₁-C₂₀ alkyl group used herein may be methyl, ethyl, propyl, isobutyl, sec-butyl, pentyl, iso-amyl, hexyl, or the like. At least one hydrogen atom of the alkyl group may be substituted with a halogen atom, a C₁-C₂₀ alkyl group substituted with a halogen atom (for example, CCF₃, CHCF₂, CH₂F, CCl₃, and the like), a hydroxyl group, a nitro group, a cyano group, an amino group, an amidino group, hydrazine, hydrozone, a carboxyl group or a salt thereof, a sulfonic acid group or a salt thereof, a phosphoric acid or a salt thereof, a C₁-C₂₀ alkyl group, a C₂-C₂₀ alkenyl group, a C₂-C₂₀ alkynyl group, a C₁-C₂₀ heteroalkyl group, a C₆-C₂₀ aryl group, a C₆-C₂₀ arylalkyl group, a C₆-C₂₀ heteroaryl group, a C₁-C₂₀ heterocyclic group, or a C₆-C₂₀ heteroarylalkyl group.

The alkoxy group used herein may be methoxy, ethoxy, or propoxy. At least one hydrogen atom of the alkoxy group may be substituted with the same substituents as in the alkyl group described above.

The C₂-C₂₀ alkenyl group used herein may be vinylene, allylene, or the like. At least one hydrogen atom of the alkenyl group may be substituted with the same substituent as in the alkyl group described above.

The unsubstituted C₂-C₂₀ alkynyl group used herein may be acetylene, or the like, and at least one hydrogen atom of the alkynyl group may be substituted with the same substituent as in the alkyl group described above.

The aryl group used herein is used alone or in combination, and refers to a C₆-C₂₀ carbocyclic aromatic system containing at least one ring, wherein the rings can be attached to each other using a pedant method or fused with each other. The term "aryl" refers to an aromatic radical, including phenyl, naphthyl, tetrahydronaphthyl, or the like. The aryl group may have a substituent such as haloalkylene, nitro, cyano, alkoxy and lower alkylamino. At least one hydrogen atom of the aryl group may be substituted with the same substituent as in the alkyl group described above.

The aryloxy group used herein may be phenoxy, and at least one hydrogen atom of the aryloxy group may be substituted with the same substituent as in the alkyl group described above.

The heteroaryl group used herein refers to a monovalent monocyclic or bicyclic aromatic bivalent organic compound which contains 1, 2 or 3 hetero atoms selected from the group consisting of N, O, P, and S, and has 1 to 20 carbon atoms. At least one hydrogen atom of the heteroaryl group may be substituted with the same substituent as in the alkyl group described above.

The cycloalkyl group used herein refers to a C₅-C₁₀ cycle group such as a cyclohexyl group, and at least one hydrogen atom of the cycloalkyl group may be substituted with the same substituent as in the alkyl group described above.

The heterocyclic group used herein refers to a 5 to 10 membered group containing a hetero atom such as nitrogen, sulfur, phosphorus, oxygen, and the like, and may be pyridyl, or the like. At least one hydrogen atom of the heterocyclic group may be substituted with the same substituent as in the alkyl group described above.

According to an embodiment of the present invention, the electrode for a fuel cell with reduced activation time and improved voltage performance according to current density and the electrolyte membrane for a fuel cell with excellent thermal stability at a high temperature and excellent capability of retaining acid can be prepared.

Hereinafter, the present invention will be described more specifically with reference to the following examples. The following examples are only for illustrative purposes and are not intended to limit the scope of the invention.

### Synthesis Example 1: Preparation of 4FPh-2AP of Formula 6 (not part of the invention)

2 g of 4-fluorophenol (17.8 mmol), 1.24 g of para-formaldehyde (39.3 mmol), and 1.85 g of 2-aminopyridine (19.6 mmol) were sequentially added to a 100 ml one-neck round bottomed flask, and then mixed in an oil bath at 90°C.

The reaction mixture was transparent in an early stage of the reaction, and about 30 minutes after the reaction, the reaction mixture was converted to a dark brown material in the form of a transparent gel. Herein, the reaction mixture was quenched with tetrahydrofurane (THF) to be cooled to room temperature.

The crude product cooled to room temperature was base washed twice by solvent extraction that used an aqueous 1 N NaOH solution, and then washed once again with deionized water.

After the washing process was terminated, an organic layer was dried using MgSO₄, and then continuously filtered. A solvent was removed from the filtrate using a rotary evaporator, and then the purified product was dried in a vacuum oven at 40°C for 6 hours.

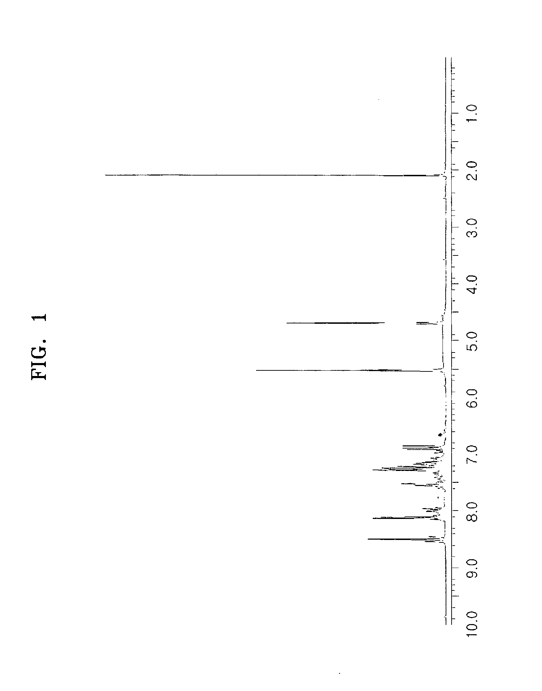

The structure of 4FPh-2AP of Formula 6 was confirmed by nuclear magnetic resonance (NMR) spectrum, and the results are shown in FIG. 1. The NMR was performed using a Varian Unity INOVA600 at 600 MHz.

### Synthesis Example 2: Preparation of 4FPh-3AP of Formula 7 (not part of the invention)

A target material was prepared in the same manner as in Synthesis Example 1, except that 3-aminopyridine was used instead of 2-aminopyridine.

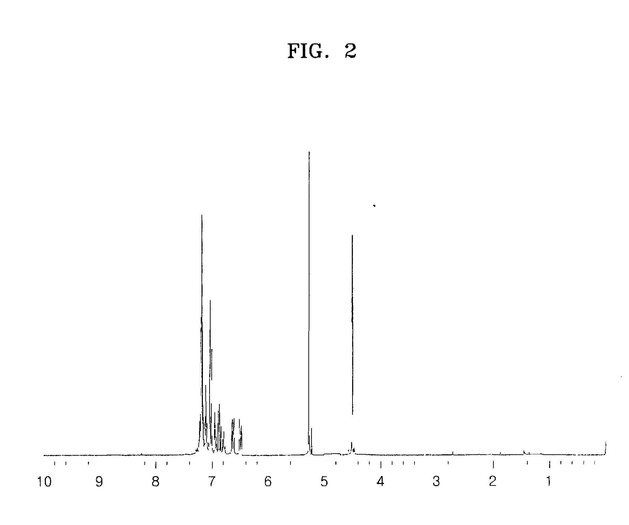

The structure of 4FPh-3AP of Formula 7 was confirmed by NMR spectrum, and the results are shown in FIG. 2. The NMR was performed using a Varian Unity INOVA600 at 600 MHz.

### Synthesis Example 3: Preparation of 34DFPh2APMD of Formula 8 (not part of the invention)

3.93 g of 3,4-difluorophenol (30 mmol), 1.98 g of para-formaldehyde (66 mmol), and 3.14 g of 2-aminopyridine (33 mmol) were sequentially added to a 100 ml one-neck round bottomed flask, and then mixed in an oil bath at 90°C.

The reaction mixture was transparent in an early stage of the reaction, and about 30 minutes after the reaction, the reaction mixture was converted to a dark brown material in the form of a transparent gel. Herein, the reaction mixture was quenched with tetrahydrofurane (THF) to be cooled to room temperature.

The crude product cooled to room temperature was base washed twice by solvent extraction that used an aqueous 1 N NaOH solution, and then washed once again with deionized water.

After the washing process was terminated, an organic layer was dried using MgSO₄, and then continuously filtered. A solvent was removed from the filtrate using a rotary evaporator, and then the purified product was dried in a vacuum oven at 40°C for 6 hours.

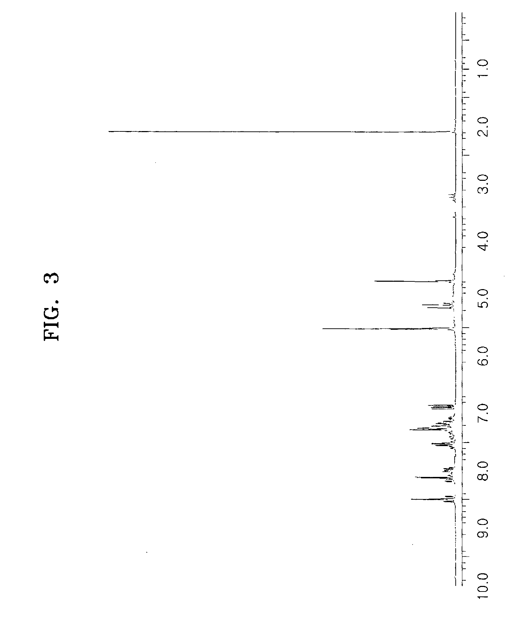

The structure of 34DFPh2APMD of Formula 8 was confirmed by NMR spectrum, and the results are shown in FIG. 3. The NMR was performed using a Varian Unity INOVA600 at 600 MHz.

### Synthesis Example 4: Preparation of 34DFPh4AP of Formula 9 (not part of the invention)

A target material was prepared in the same manner as in Synthesis Example 3, except that 3.1 g of 4-aminopyridine (33 mmol) was used instead of 3.14 g of 2-aminopyridine (33 mmol).

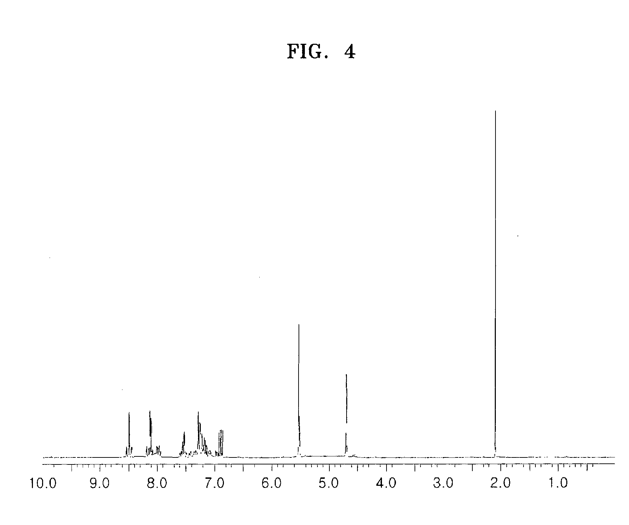

The structure of 34DFPh4AP of Formula 9 was confirmed by NMR spectrum, and the results are shown in FIG. 4. The NMR was performed using a Varian Unity INOVA600 at 600 MHz.

### Synthesis Example 5: Preparation of 3HP-34DFA of Formula 10 (not part of the invention)

A target material was prepared in the same manner as in Synthesis Example 1, except that 2 g of 3-hydroxypyridine (21 mmol), 1.46 g of para-formaldehyde (46.3 mmol), and 2.98 g of 3,4-difluoroaniline (23.1 mmol) were added to a 100 ml one-neck round bottomed flask.

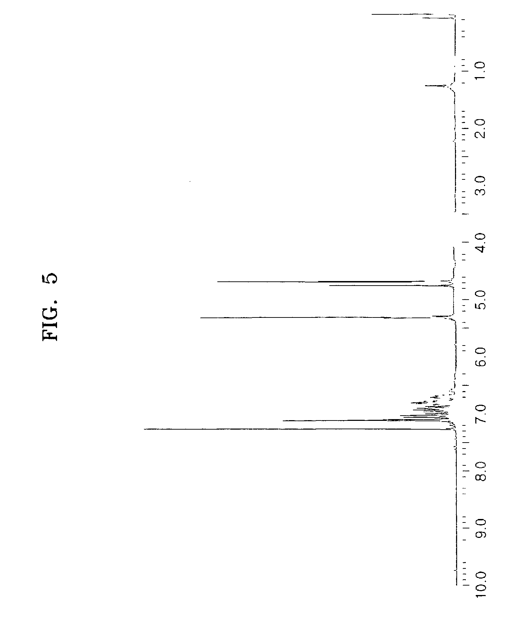

The structure of 3HP-34DFA of Formula 10 was confirmed by NMR spectrum, and the results are shown in FIG. 5. The NMR was performed using a Varian Unity INOVA600 at 600 MHz.

### Synthesis Example 6: Preparation of 8HQ-34DFA of Formula 11

A target material was prepared in the same manner as in Synthesis Example 5, except that alcohol represented by the following formula was used instead of 2 g of 3-hydroxypyridine (21 mmol).

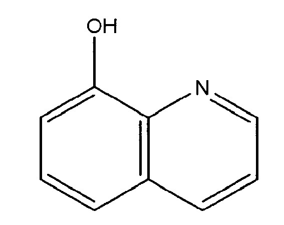

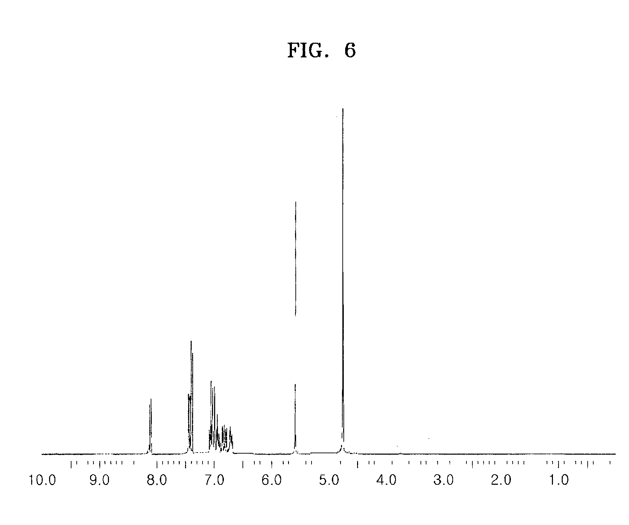

The structure of 8HQ-34DFA of Formula 11 was confirmed by NMR spectrum, and the results are shown in FIG. 6. The NMR was performed using a Varian Unity INOVA600 at 600 MHz.

### Synthesis Example 7: Preparation of 8HQD-34DFA of Formula 12

A target material was prepared in the same manner as in Synthesis Example 5, except that alcohol represented by the following formula was used instead of 2 g of 3-hydroxypyridine (21 mmol).

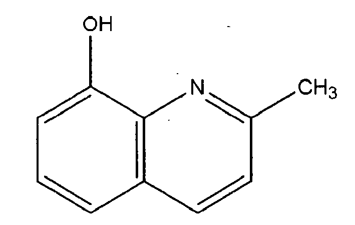

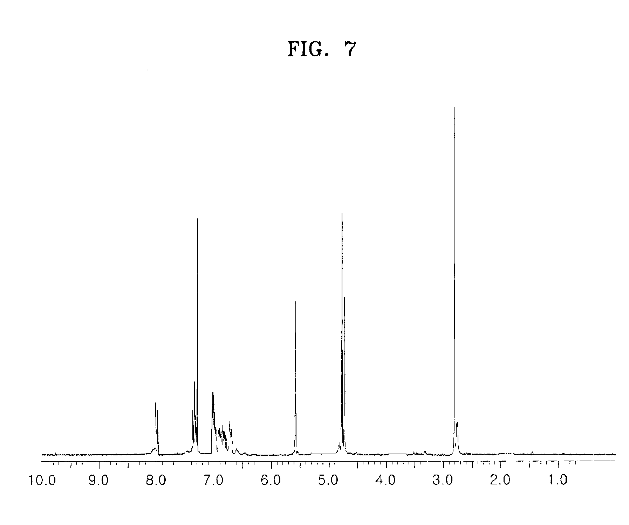

The structure of 8HQD-34DFA of Formula 12 was confirmed by NMR spectrum, and the results are shown in FIG. 7. The NMR was performed using a Varian Unity INOVA600 at 600 MHz.

### Synthesis Example 8: Preparation of polymer of 4FPh-2AP of Formula 6 (not part of the invention)

20 g of 4FPh-2AP of Formula 6 and dimethylacetamide were mixed together, and then the mixture was cured at about 220°C to obtain a polymer of 4FPh-2AP of Formula 6.

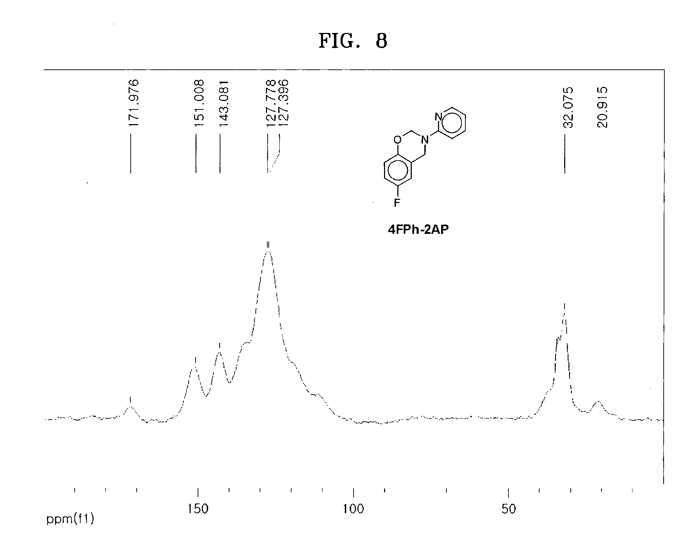

The structure of the solid-phase polymer of 4FPh-2AP of Formula 6 was identified by a solid nuclear magnetic resonance (NMR) spectrum, and the results are shown in FIG. 8. The NMR was performed using a Varian Unity INOVA600 at 600 MHz.

### Reference Example 1: Preparation of t-BuPh-a

15 g of t-butylphenol (0.1 mol), 6.31 g of para-formaldehyde (0.21 mol), and 10.24 g of aniline (0.11 mol) were sequentially added in a 100 ml one-neck round bottom flask, and then mixed in an oil bath at 90°C.

The reaction mixture was opaque in an early stage of the reaction, and about 30 minutes after the reaction, the reaction mixture was converted to a dark brown material in the form of a transparent gel. At this time, the reaction mixture was quenched with tetrahydrofurane (THF) to be cooled to room temperature.

The crude product cooled to room temperature was base washed twice by solvent extraction that used an aqueous 1 N NaOH solution, and then washed once again with deionized water. After the washing process was terminated, an organic layer was dried using MgSO₄, and then continuously filtered. The solvent was removed from the filtered solution using a rotary evaporator, and then the purified product was dried in a vacuum oven at 40°C for 6 hours to obtain t-BuPh-a.

Thermal stabilities of 3HP-34DFA of Synthesis Example 5 and t-BuPh-a were evaluated using thermogravimetric analysis (TGA). FIG. 1 is a graph showing thermogravimetric analysis (TGA) results of 4FPh-2AP prepared in Synthesis Example 5 and t-BuPh-a prepared in Reference Example 1.

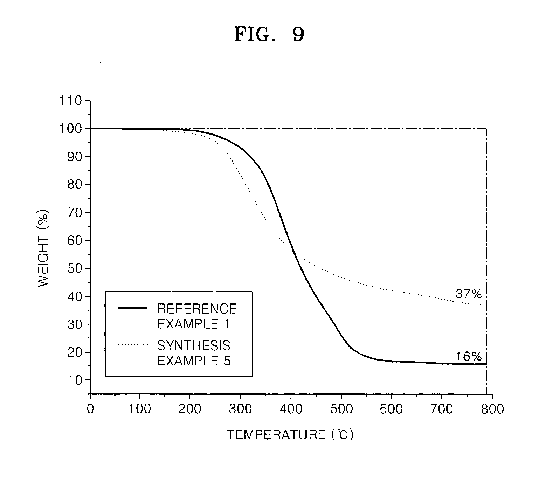

Thermal stabilities of 3HP-34DFA prepared in Synthesis Example 5 and t-BuPh-a prepared in Reference Example 1 were evaluated using thermogravimetric analysis (TGA). The results are shown in FIG. 9. In FIG. 9, thermogravimetric loss was measured at 800°C.

Referring to FIG.9, it was confirmed that 3HP-34DFA of Synthesis Example 5 had less thermogravimetric loss at a temperature of 800°C or more than that of t-BuPh-a. From the result, it can be seen that 3HP-34DFA of Synthesis Example 5 has excellent thermal stability compared to t-BuPh-a.

### Example 1: Preparation of electrode for fuel cell and fuel cell including the electrode

1 g of a catalyst in which 50 wt% of PtCo was supported on carbon and 3 g of NMP were added to a stirrer, and the mixture was stirred using a mortar to prepare a slurry. A NMP solution of 4FPh-2AP of Synthesis Example 1 was added to the slurry in order that the resultant contained 0.025 g of 4FPh-2AP of Formula 6. The resultant was further stirred.

Subsequently, a NMP solution of 5 wt% of polyvinylidenefluoride was added to the resultant to make the resultant contain 0.025 g of polyvinylidenefluoride. The resultant was mixed for 10 minutes to prepare a slurry used for forming a cathode catalyst layer.

Carbon paper was cut to a size of 4x7 cm², fixed on a glass plate, and coated by a doctor blade (Sheen instrument). Herein, a gap interval was adjusted to 600 µm.

The slurry used for forming a cathode catalyst layer was coated on the carbon paper, and the resultant was dried at room temperature for 1 hour, dried at 80°C for 1 hour, dried at 120°C for 30 minutes, and dried at 150°C for 15 minutes to prepare a cathode (a fuel electrode). The loading amount of PtCo in the prepared cathode was 2.1 mg/cm².

An electrode prepared by the following processes was used as an anode.

2 g of a catalyst in which 50 wt% of Pt was supported on carbon and 9 g of NMP were added to a stirrer, and the mixture was stirred for 2 minutes using a high speed stirrer.

Subsequently, a solution in which 0.05 g of polyvinylidenefluoride was dissolved in 1 g of NMP was added to the mixture, and the resultant was further stirred for 2 minutes to prepare a slurry used for forming an anode catalyst layer. The slurry used for forming an anode catalyst layer was coated on carbon paper coated with a microporous layer using a bar coater. As a result, preparation of the anode was completed. The loading amount of Pt in the prepared anode was 1.3 mg/cm².

Separately, 60 parts by weight of the benzoxazine-based monomer of Formula A, 3 parts by weight of the benzoxazine-based monomer of Formula B where R₂ was a phenyl group, and 37 parts by weight of polybenzimidazole were blended together, and then the mixture was cured at about 220°C.

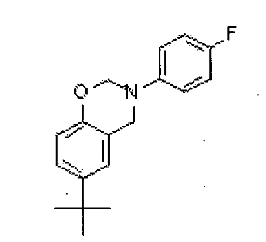

### Benzoxazine-based monomer A

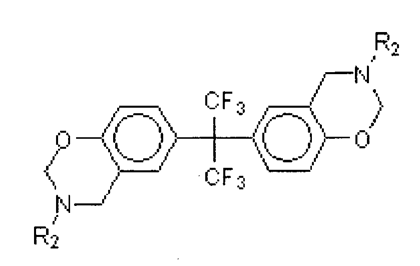

wherein R₂ is a phenyl group

### Benzoxazine-based monomer B

Subsequently, the resultant was impregnated with 85 wt% of phosphoric acid at 80°C for over 4 hours to form an electrolyte membrane. Herein, the amount of phosphoric acid was about 480 parts by weight based on 100 parts by weight of the total weight of the electrolyte membrane.

The electrolyte membrane was disposed between the cathode and the anode to prepare a MEA. Herein, the cathode and anode were not impregnated with phosphoric acid.

To prevent gas permeation between the cathode and the anode, a teflon membrane for a main gasket with a thickness of 200 µm and a teflon membrane for a subgasket with a thickness of 20 µm were joined and disposed between the electrode and the electrolyte membrane.

Electricity was generated by causing hydrogen to flow into the anode (flowrate: 100 cc/m) and causing air to flow into the cathode (flowrate: 250 cc/m) at 150°C under a condition where the electrolyte membrane was not humidified, and properties of a fuel cell prepared were measured. Herein, an electrolyte doped with a phosphoric acid was used, and thus the performance of the fuel cell improved as time elapsed. Thus, aging was performed until an operating voltage reached a peak, and then the properties of the fuel cell were finally evaluated. In addition, the area of the cathode and anode was fixed to a size of 2.8x2.8 (7.84 cm²), and the thickness of the cathode was about 430 µm and the thickness of the anode was about 390 µm.

### Example 2-6 : Preparation of electrode for fuel cell and fuel cell including the electrode

Cathodes were prepared in the same manner as in Example 1, except that 4FPh-3AP of Synthesis Example 2, 34DFPh2APMD of Synthesis Example 3, 34DFPh4AP of Synthesis Example 4, 3HP-34DFA of Synthesis Example 5, and 8HQ-34DFA of Synthesis Example 6, respectively were used instead of 4FPh-2AP of Synthesis Example 1, and fuel cells using the cathodes were manufactured.

### Comparative Example 1 : Preparation of electrode for fuel cell and fuel cell including the electrode

A cathode was prepared in the same manner as in Example 1, except that 4FPh2AP of Formula 2 of Synthesis Example 1 was not used, and a fuel cell using the cathode was manufactured.

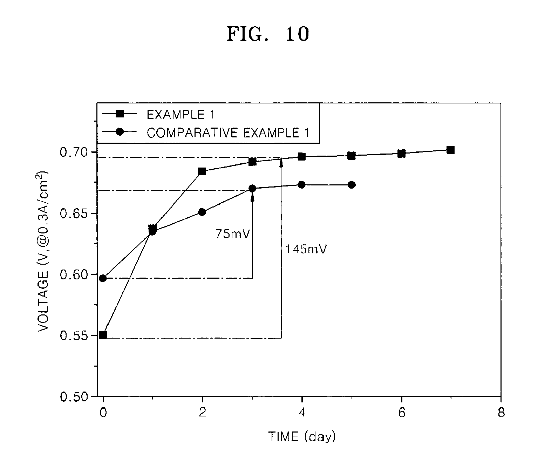

FIG. 10 is a graph showing a change in voltage with respect to time of fuel cells manufactured in Example 1 and Comparative Example 1.

Referring to FIG. 10, although the fuel cell of Example 1 had low initial performance, it had improved voltage performance by fast activation compared to the fuel cell of Comparative Example 1.

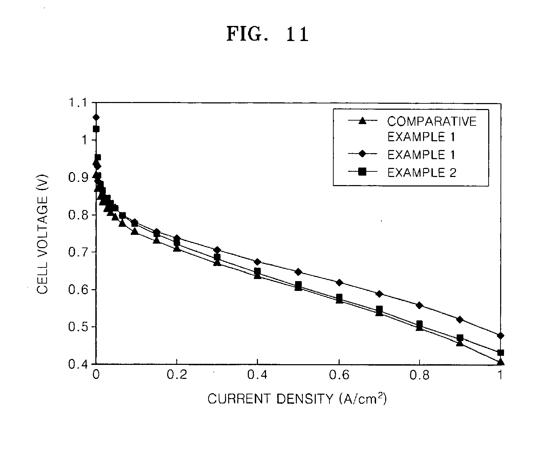

In addition, a change in cell potential with respect to current density of the fuel cells of Examples 1 and 2 and Comparative Example 1 was measured, and the results are shown in FIG. 11. FIG. 11 is a graph showing a change in cell voltage with respect to current density of the fuel cells manufactured in Examples 1 and 2 and Comparative Example 1.

Referring to FIG. 11, the fuel cells of Examples 1 and 2 had improved cell voltage characteristics with respect to current density compared to the fuel cell of Comparative Example 1.

### Example 7: Preparation of electrolyte membrane for fuel cell and fuel cell using the electrolyte membrane

1 g of a catalyst in which 50 wt% of PtCo was supported on carbon and 3 g of NMP as a solvent were added to a stirrer, and the mixture was stirred using a mortar to prepare a slurry. A NMP solution of 4FPh-2AP of Formula 6 of Synthesis Example 1 was added to the slurry in order that the resultant contained 0.025 g of 4FPh-2AP of Formula 6. The resultant was further stirred.

Subsequently, a NMP solution of 5 wt% of polyvinylidenefluoride was added to the resultant to make the resultant contain 0.025 g of polyvinylidenefluoride. The resultant was mixed for 10 minutes to prepare a slurry used for forming a cathode catalyst layer.

Carbon paper was cut to a size of 4x7 cm², fixed on a glass plate, and coated by a doctor blade (Sheen instrument). Herein, a gap interval was adjusted to 600 µm.

The slurry used for forming a cathode catalyst layer was coated on the carbon paper, and the resultant was dried at room temperature for 1 hour, dried at 80°C for 1 hour, dried at 120°C for 30 minutes, and dried at 150°C for 15 minutes to prepare a cathode (a fuel electrode). The loading amount of PtCo in the prepared cathode was 2.32 mg/cm².

An electrode prepared by the following processes was used as an anode.

2 g of a catalyst in which 50 wt% of Pt was supported on carbon and 9 g of NMP as a solvent were added to a stirrer, and the mixture was stirred for 2 minutes using a high speed stirrer.

Subsequently, a solution in which 0.05 g of polyvinylidenefluoride was dissolved in 1 g of NMP was added to the mixture, and the resultant was further stirred for 2 minutes to prepare a slurry used for forming an anode catalyst layer. The slurry used for forming an anode catalyst layer was coated on carbon paper coated with a microporous layer using a bar coater. As a result, preparation of the anode was completed. The loading amount of Pt in the prepared anode was 1.44 mg/cm².

Separately, 65 parts by weight of 4FPh-2AP of Formula 6 of Synthesis Example 1, and 35 parts by weight of polybenzimidazole (PBI) represented by the following formula were blended together, and then the mixture was cured at about 220 °C.

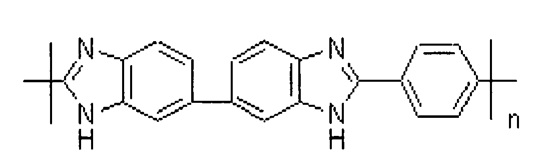

A weight average molecular weight of polybenzimidazole was about 20,000.

Subsequently, the resultant was impregnated with 85 wt% of phosphoric acid at 80°C for over 4 hours to form an electrolyte membrane. Herein, the amount of phosphoric acid was about 500 parts by weight based on 100 parts by weight of the total weight of the electrolyte membrane.

The electrolyte membrane was disposed between the cathode and the anode to prepare a MEA. Herein, the cathode and anode were not impregnated with phosphoric acid.

To prevent gas permeation between the cathode and the anode, a teflon membrane for a main gasket with a thickness of 200 µm and a teflon membrane for a subgasket with a thickness of 20 µm were joined and disposed between the electrode and the electrolyte membrane. The pressure applied to the MEA was adjusted to 1, 2, 3 N-m Torque step by step using a wrench to assemble a cell.

Electricity was generated by causing hydrogen to flow into the anode (flowrate: 100 cc/m) and causing air to flow into the cathode (flowrate: 250 cc/m) at 150°C under a condition where the electrolyte membrane was not humidified, and properties of a fuel cell prepared were measured. Herein, an electrolyte doped with a phosphoric acid was used, and thus the performance of the fuel cell improved as time elapsed. Thus, aging was performed until an operating voltage reached a peak, and then the properties of the fuel cell were finally evaluated. In addition, the area of the cathode and anode was fixed to a size of 2.8×2.8 (7.84 cm²), and the thickness of the cathode was about 430 µm and the thickness of the anode was about 390 µm.

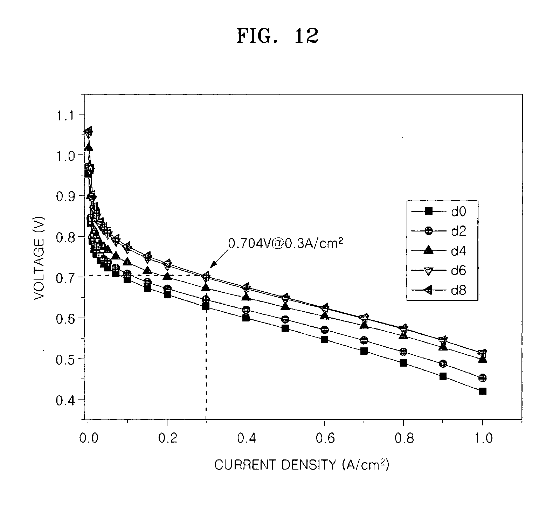

Cell voltage according to current density of the fuel cell manufactured in Example 7 was measured, and the results are shown in FIG. 12. FIG. 12 is a graph showing results of cell voltage according to current density of the fuel cell of Example 7 after d0, d2, d4, d6, and d8, wherein d0 denotes right after operation, d2 denotes after 2 days, d4 denotes after 4 days, d6 denotes after 6 days, and d8 denotes after 8 days.

Referring to FIG. 12, the fuel cell of Example 7 have increased cell voltage according to operating time.

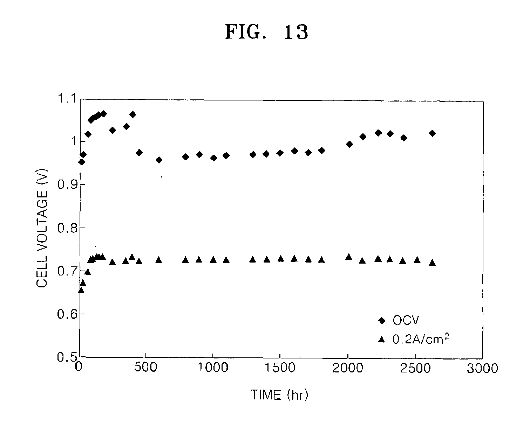

Durability of the electrolyte membrane prepared in Example 7 was measured, and the results are shown in FIG. 13. In FIG. 13, , "OCV" denotes an open circuit voltage, and "0.2 A/cm²" denotes cell voltage at a current density of 0.2 A/cm².

Referring to FIG. 13, electrolyte membrane prepared in Example 7 shows nearly no voltage drop until 2500 hours.

### Example 8: Preparation of electrolyte membrane for fuel cell and fuel cell using the electrolyte membrane

An electrolyte membrane and a fuel cell using the electrolyte membrane were prepared in the same manner as in Example 7, except that 3HP-34DFA represented by Formula 10 was used instead of 4FPh-2AP of Formula 6 in the formation of the electrolyte membrane.

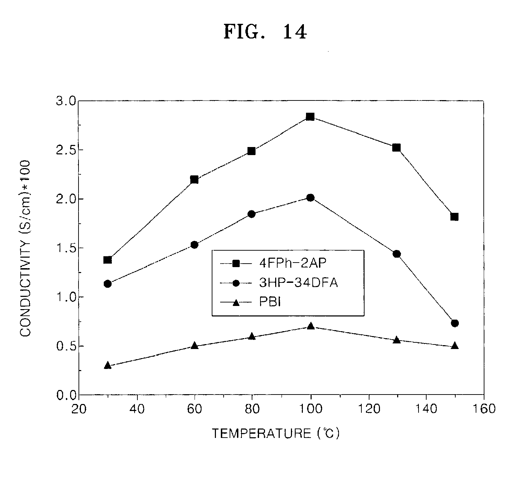

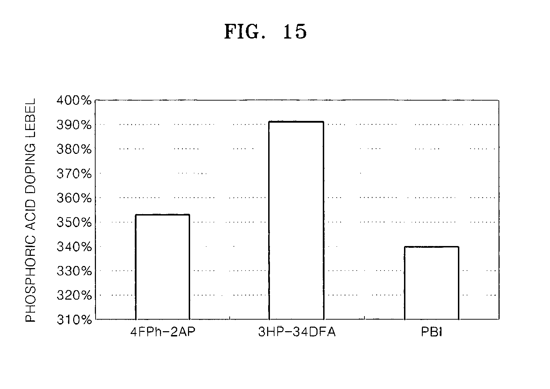

Conductivity according to temperature and phosphoric acid doping level of the electrolyte membranes prepared in Examples 7 and 8 were measured, and the results are respectively shown in FIGS. 14 and 15.

Referring to FIGS. 14 and 15, the electrolyte membranes of Examples 7 and 8 have higher ionic conductivity and excellent durability.

Referring to FIG. 15, the electrolyte membranes of Examples 7 and 8 have higher conductivity compared with the PBI electrolyte membrane.

In FIG. 15, the phosphoric acid doping level of 4FPh-2AP is 352%. This represents that the doping level is 352 parts by weight of phosphoric acid based on 100 parts by weight of 4FPh-2AP.

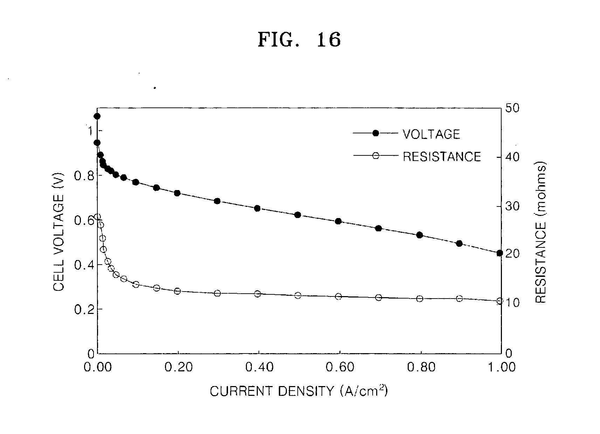

In addition, a change in voltage and resistance according to current density of the fuel cell manufactured in Example 8 was measured, and the results are shown in FIG. 16. Referring to FIG. 16, the fuel cell of Example 8 has excellent voltage and resistance characteristics.

### Example 9: Preparation of electrolyte membrane for fuel cell and fuel cell using the electrolyte membrane

An electrolyte membrane and a fuel cell using the electrolyte membrane were prepared in the same manner as in Example 7, except that 3HQ-34DFA represented by Formula 11 was used instead of 4FPh-2AP of Formula 6 in the formation of the electrolyte membrane.

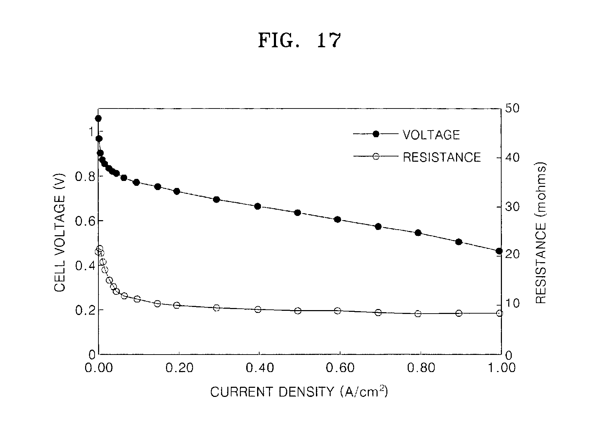

A change in cell voltage and resistance according to current density of the fuel cell of Example 9 was measured, and the results are shown in FIG. 17.

Referring to FIG. 17, the fuel cell manufactured in Example 9 has excellent cell voltage and resistance characteristics.

### Example 10: Preparation of electrolyte membrane for fuel cell and fuel cell using the electrolyte membrane

An electrolyte membrane and a fuel cell using the electrolyte membrane were prepared in the same manner as in Example 7, except that 3HQD-34DFA represented by Formula 12 was used instead of 4FPh-2AP of Formula 6 in the formation of the electrolyte membrane.

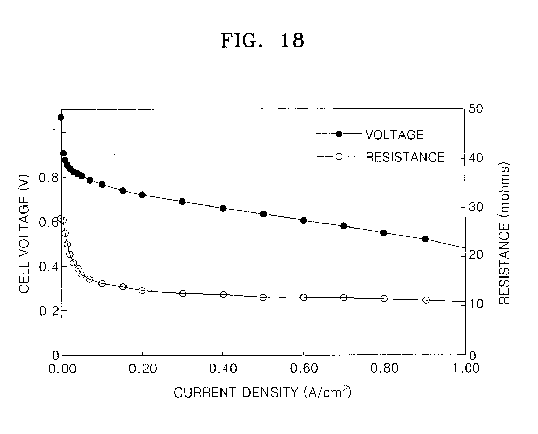

A change in cell voltage and resistance according to current density of the fuel cell of Example 10 was measured, and the results are shown in FIG. 18.

Referring to FIG. 18, the fuel cell manufactured in Example 10 has excellent cell voltage and resistance characteristics as in the fuel cell of Example 7.

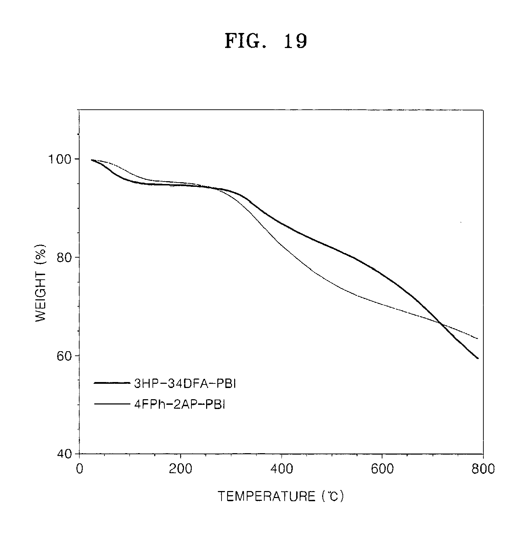

Thermal stabilities of the electrolyte membranes prepared in Examples 7 and 8 were evaluated using thermogravimetric analysis (TGA). The results are shown in FIG. 19. In FIG. 19, thermogravimetric loss was measured at 800°C.

Referring to FIG. 19, the electrolyte membranes of Examples 7 and 8 have excellent thermal stability.

### Example 11: Preparation of fuel cell

1 g of a catalyst in which 50 wt% of PtCo was supported on carbon and 3 g of NMP as a solvent were added to a stirrer, and the mixture was stirred using a mortar to prepare a slurry. A NMP solution of 4FPh-2AP of Formula 6 was added to the slurry in order that the resultant contained 0.025 g of 4FPh-2AP of Formula 6. The resultant was further stirred.

Subsequently, a NMP solution of 5 wt% of polyvinylidenefluoride was added to the resultant to make the resultant contain 0.025 g of polyvinylidenefluoride. The resultant was mixed for 10 minutes to prepare a slurry used for forming a cathode catalyst layer.

Carbon paper was cut to a size of 4x7 cm², fixed on a glass plate, and coated by a doctor blade (Sheen instrument). Herein, a gap interval was adjusted to 600 µm.

The slurry used for forming a cathode catalyst layer was coated on the carbon paper, and the resultant was dried at room temperature for 1 hour, dried at 80°C for 1 hour, dried at 120°C for 30 minutes, and dried at 150°C for 15 minutes to prepare a cathode (a fuel electrode). The loading amount of PtCo in the prepared cathode was 2.03 mg/cm².

An electrode prepared by the following processes was used as an anode.

2 g of a catalyst in which 50 wt% of Pt was supported on carbon and 9 g of NMP as a solvent were added to a stirrer, and the mixture was stirred for 2 minutes using a high speed stirrer.

Subsequently, a solution in which 0.05 g of polyvinylidenefluoride was dissolved in 1 g of NMP was added to the mixture, and the resultant was further stirred for 2 minutes to prepare a slurry used for forming an anode catalyst layer. The slurry used for forming an anode catalyst layer was coated on carbon paper coated with a microporous layer using a bar coater. As a result, preparation of the anode was completed. The loading amount of Pt in the prepared anode was 1.34 mg/cm².

Separately, 65 parts by weight of 4FPh-2AP of Formula 6 and 35 parts by weight of polybenzimidazole were blended together, and then the mixture was cured at about 220°C.

Then, the resultant was impregnated with 85% by weight of phosphoric acid at 80°C for longer than 4 hours to prepare an electrolyte membrane. In this regard, the amount of phosphoric acid was about 440 parts by weight based on 100 parts by weight of electrolyte membrane.

A membrane electrode assembly (MEA) was prepared by interposing the electrolyte membrane between the cathode and the anode. In this regard, the cathode and anode were not impregnated with phosphoric acid.

A 200 µm teflon membrane for a main gasket and a 20 µm teflon membrane for a sub gasket were overlapped on an interface between the electrodes and electrolyte membrane in order to prevent gas permeation between the cathode and the anode. The pressure applied to the MEA was adjusted to 1, 2, 3 N-m Torque step by step using a wrench to assemble a cell.

Characteristics of fuel cells were measured while operating by supplying hydrogen to the anode at 100 cc/m and supplying air to the cathode at 250 cc/m while the electrolyte membrane was not hydrated at 150°C. Since cell efficiency increases with time by using the electrolyte doped with phosphoric acid, the final efficiency was measured after the fuel cell is aged until operational voltage was maximized. The area of the cathode and the anode is fixed to 2.8x2.8=7.84 cm², and the thickness of the cathode was about 430 µm and the thickness of the anode was about 390 µm although the thicknesses of the cathode and the anode may vary according to distribution of carbon paper.

### Comparative Example 2: Preparation of fuel cell

A fuel cell was prepared in the same manner as in Example 11, except that a polybenzimidazole (PBI) membrane was used as an electrolyte membrane and 4FPh2AP of Formula 6 was not used in the preparation of the cathode.

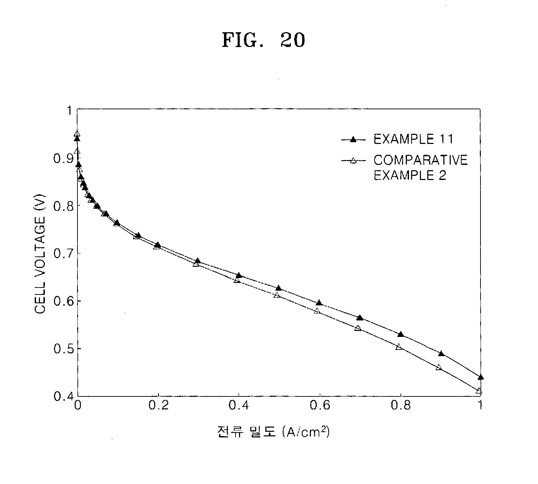

Cell voltage characteristics with respect to current density of the fuel cells prepared in Example 11 and Comparative Example 2 were measured, and the results are shown in FIG. 20.

Referring to FIG. 20, cell voltage of the fuel cell of Example 11 is improved compared with that of the fuel cell of Comparative Example 2.

While aspects of the present invention have been particularly shown and described with reference to differing embodiments thereof, it should be understood that these exemplary embodiments should be considered in a descriptive sense only and not for purposes of limitation. Descriptions of features or aspects within each embodiment should typically be considered as available for other similar features or aspects in the remaining embodiments.

Thus, although a few embodiments have been shown and described, it would be appreciated by those of ordinary skill in the art that changes may be made in these embodiments without departing from the principles of the invention, the scope of which is defined in the claims and their equivalents.

## Claims

1. A benzoxazine-based monomer represented by Formula 5 or Formula 12 below: 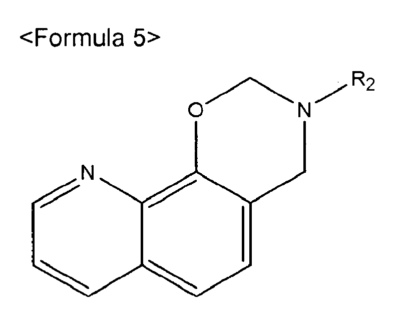 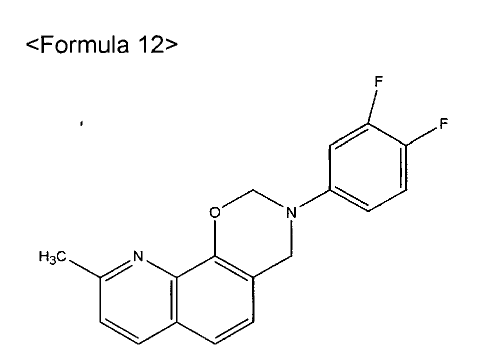 wherein R₂ in Formula 5 is one of the groups represented by the following formulae.

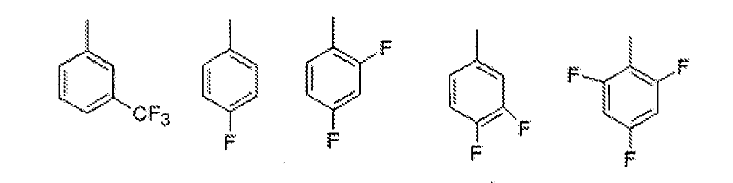

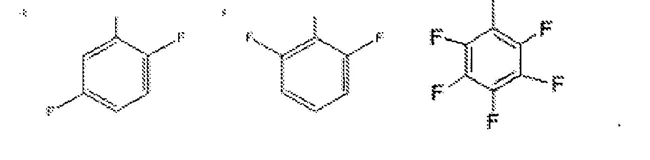

2. The benzoxazine-based monomer of claim 1, which is a compound represented by Formula 11 below:

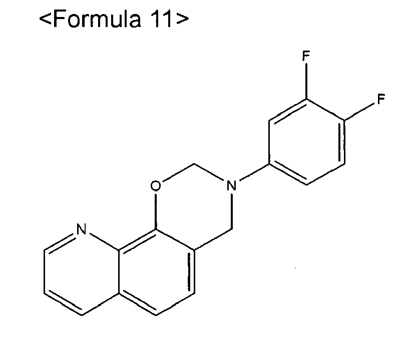

3. A polymer of benzoxazine -based monomer which is a polymerization product of the benzoxazine -based monomer according to claim 1 or 2 or a polymerization product of the benzoxazine-based monomer according to claim 1 or 2 and a crosslinkable compound.

4. An electrode for a fuel cell comprising a catalyst layer comprising the polymer of the benzoxazine -based monomer of claim 3.

5. The electrode of claim 4, wherein the catalyst of the catalyst layer is a catalyst metal or a support catalyst in which the catalyst metal is loaded on a carbonaceous support,
wherein the catalyst metal is:
Pt;
a metal-Pt alloy including Pt and at least one metal selected from the group consisting ofAu, Pd, Rh, Ir, Ru, Sn, Mo, Co, and Cr; or
a mixture including Pt and at least one metal selected from the group consisting of Au, Pd, Rh, Ir, Ru, Sn, Mo, Co, and Cr.

6. The electrode of claim 4 or 5, wherein the catalyst layer further comprises at least one proton conductor selected from the group consisting of a phosphoric acid and a C₁-C₂₀ organic phosphonic acid.

7. An electrolyte membrane for a fuel cell comprising the polymer of the benzoxazine -based monomer of claim 3.

8. A fuel cell comprising: a cathode; an anode; and an electrolyte membrane interposed between the cathode and the anode,
wherein at least one of the cathode and the anode is an electrode according to any of claims 4-6, and/or the electrolyte membrane is an electrolyte membrane according to claim 7.

## Patentansprüche

1. Ein Benzoxazin-basiertes Monomer, dargestellt durch untenstehende Formel 5 oder Formel 12: 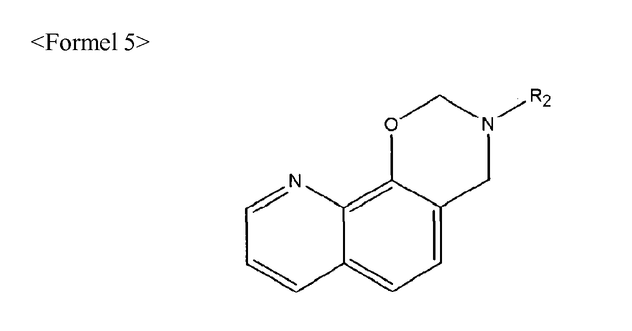 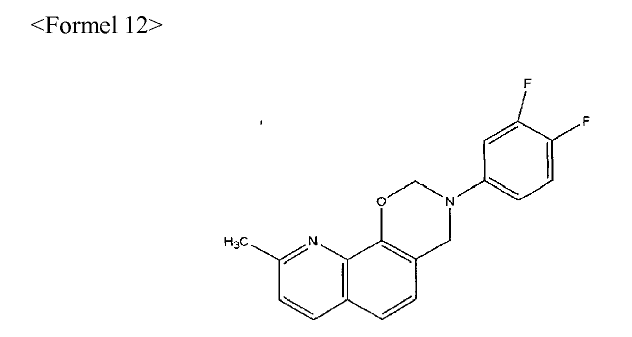 wobei R₂ in Formel 5 eines aus der Gruppe, dargestellt durch die folgenden Formeln, ist.

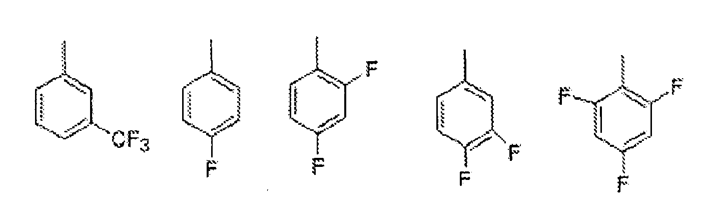

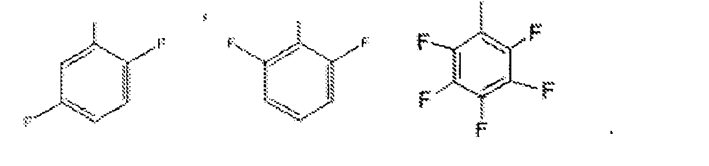

2. Das Benzoxazin-basierte Monomer von Anspruch 1, das eine Verbindung ist, die durch untenstehende Formel 11 dargestellt wird:

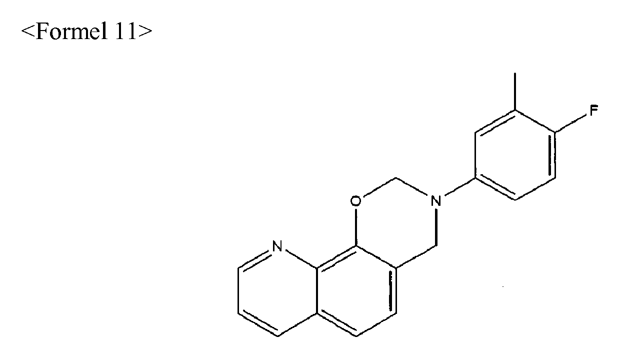

3. Ein Polymer eines Benzoxazin-basierten Monomers, das ein Polymerisationsprodukt des Benzoxazin-basierten Monomers gemäß Anspruch 1 oder 2 oder ein Polymerisationsprodukt des Benzoxazin-basierten Monomers gemäß Anspruch 1 oder 2 und eine vemetzungsfähige Verbindung ist.

4. Eine Elektrode für eine Brennstoffzelle, umfassend eine Katalysatorschicht, die das Polymer des Benzoxazin-basierten Monomers von Anspruch 3 umfasst.

5. Die Elektrode von Anspruch 4, wobei der Katalysator der Katalysatorschicht ein Katalysatormetall oder ein unterstützender Katalysator ist, in dem das Katalysatormetall auf einen kohlenstoffhaltigen Träger geladen wird,
wobei das Katalysatormetall Folgendes ist:
Pt;
eine Metall-Pt-Legierung, die Pt und mindestens ein Metall beinhaltet, das ausgewählt wird aus der Gruppe bestehend aus Au, Pd, Rh, Ir, Ru, Sn, Mo, Co und Cr; oder
eine Mischung, die Pt und mindestens ein Metall beinhaltet, das ausgewählt wird aus der Gruppe bestehend aus Au, Pd, Rh, Ir, Ru, Sn, Mo, Co und Cr.

6. Die Elektrode von Anspruch 4 oder 5, wobei die Katalysatorschicht ferner mindestens einen Protonenleiter umfasst, der ausgewählt wird aus der Gruppe bestehend aus einer Phosphorsäure und einer organischen C₁-C₂₀-Phosphonsäure.

7. Eine Elektrolytmembran für eine Brennstoffzelle, umfassend das Polymer des Benzoxazin-basierten Monomers von Anspruch 3.

8. Eine Brennstoffzelle, umfassend: eine Kathode; eine Anode und eine Elektrolytmembran, die zwischen der Kathode und der Anode eingefügt ist,
wobei mindesten eine von der Kathode und der Anode eine Elektrode gemäß einem der Ansprüche 4-6 ist und/oder die Elektrolytmembran eine Elektrolytmembran gemäß Anspruch 7 ist.

## Revendications

1. Monomère à base de benzoxazine représenté par la formule 5 ou la formule 12 ci-après : 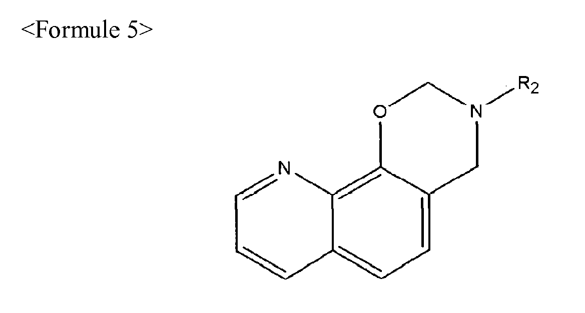 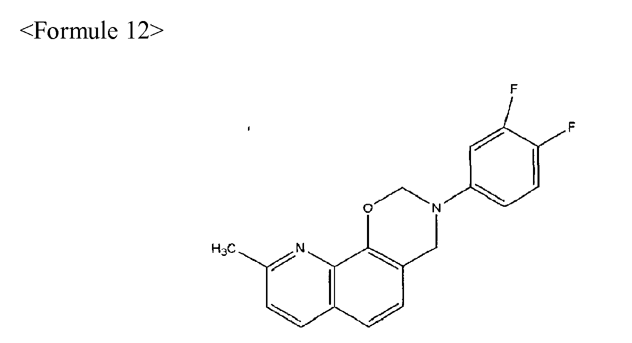 dans lequel R₂ est dans la formule 5 un des groupes représentés par les formules suivantes.

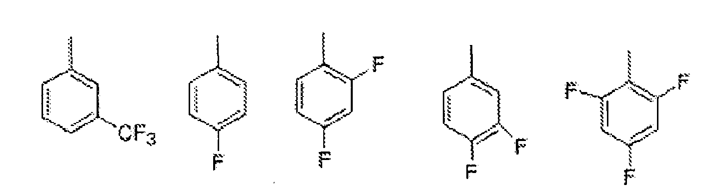

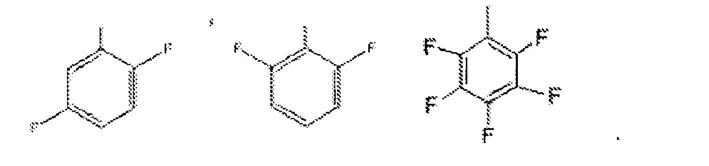

2. Monomère à base de benzoxazine selon la revendication 1, qui est un composé représenté par la formule 11 ci-après :

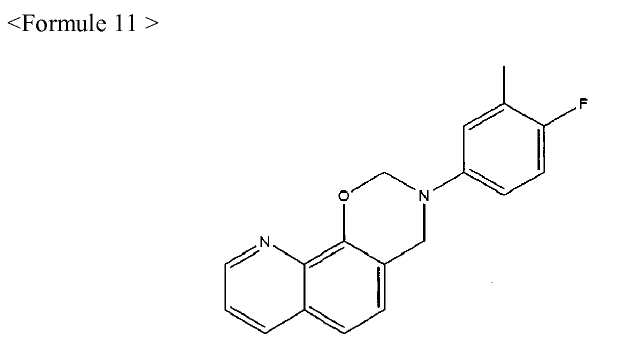

3. Polymère de monomère à base de benzoxazine qui est un produit de polymérisation du monomère à base de benzoxazine selon la revendication 1 ou 2 ou un produit de polymérisation du monomère à base de benzoxazine selon la revendication 1 ou 2 et un composé apte à une liaison croisée.

4. Electrode pour cellule de combustible comprenant une couche de catalyse comprenant le polymère de monomère à base de benzoxazine selon la revendication 3.

5. Electrode selon la revendication 4, dans laquelle le catalyseur de la couche de catalyse est un métal de catalyse ou un catalyseur support dans lequel le métal de catalyse est chargé sur un support carboné,
dans laquelle le métal de catalyse est :
du Pt;
un alliage métallique de Pt contenant du Pt et au moins un métal sélectionné dans le groupe composé de : Au, Pd, Rh, Ir, Ru, Sn, Mo, Co, et Cr ; ou
un mélange contenant du Pt et au moins un métal sélectionné dans le groupe composé de : Au, Pd, Rh, Ir, Ru, Sn, Mo, Co, et Cr.

6. Electrode selon la revendication 4 ou 5, dans laquelle la couche de catalyse comprend en outre au moins un conducteur de protons sélectionné dans le groupe composé de :
un acide phosphorique et un acide phosphonique organique C1-C20.

7. Membrane à électrolyte pour cellule de combustible comprenant le polymère de monomère à base de benzoxazine benzoxazine selon la revendication 3.

8. Cellule de combustible comprenant : une cathode; une anode ; et une membrane à électrolyte intercalée entre la cathode et l'anode,
dans laquelle au moins une des deux sur la cathode et l'anode est une électrode selon l'une quelconque des revendications 4 à 6, et/ou la membrane à électrolyte est une membrane à électrolyte selon la revendication 7.
